# EUROPEAN PATENT SPECIFICATION

(11) **EP 1 457 772 B1**
(45) Date of publication and mention of the grant of the patent: **01.12.2010**
(21) Application number: 04075840.1
(22) Date of filing: 12.03.2004
(51) Int. Cl.: G01N 27/12, G01N 33/00

(54) **Gas sensor comprising a resistance dependent organic-inorganic intercalated hybrid material and manufacturing method of said sensor**
Gassensor, enthaltend ein widerstandsabhängiges organisch-inorganisches interkaliertes Hybridmaterial und Methode zur Herstellung des besagten Sensors
Capteur de gaz comprenant un matériau hybride organique-inorganique intercalé dépendant de la résistance et méthode pour la production de ce capteur

(30) Priority: 12.03.2003 JP 2003066108
(43) Date of publication of application: 15.09.2004
(73) Proprietor: National Institute of Advanced Industrial Science and Technology, Tokyo 100-8921 (JP)
(72) Inventor: Matsubara, Ichiro, c/o National Institute of Tech, Moriyama-ku, Nagoya-shi, Aichi 463-8560 (JP); Murayama, Norimitsu, c/o National Instit of Tech, Moriyama-ku, Nagoya-shi, Aichi 463-8560 (JP); Shin, Woosuck, c/o National Instit of Tech, Moriyama-ku, Nagoya-shi, Aichi 463-8560 (JP); Izu, Noriya, c/o National Instit of Advanced Tech, Moriyama-ku, Nagoya-shi, Aichi 463-8560 (JP); Hosono, Kouta, c/o National Instit of Tech, Moriyama-ku, Nagoya-shi, Aichi 463-8560 (JP)
(74) Representative: van Westenbrugge, Andries

(56) References cited:
- US-B1- 6 400 489
- BIZETO M A ET AL: "PORPHYRIN INTERCALATION INTO A LAYERED NIOBATE DERIVED FROM K4NB6O17" JOURNAL OF MATERIALS SCIENCE, CHAPMAN AND HALL LTD. LONDON, GB, vol. 37, no. 2, 15 January 2002 (2002-01-15), pages 265-270, XP001092493 ISSN: 0022-2461
- GOMEZ-ROMERO P ET AL: "HYBRID ORGANIC-INORGANIC ELECTRODES: THE MOLECULAR MATERIAL FORMED BETWEEN POLYPYRROLE AND THE PHOSPHOMOLYBDATE ANION" ADVANCED MATERIALS, VCH VERLAGSGESELLSCHAFT, WEINHEIM, DE, vol. 9, no. 2, 1 February 1997 (1997-02-01), pages 144-147, XP000681083 ISSN: 0935-9648
- GOPALAKRISHNAN J ET AL: "Polymerization of aniline in layered perovskites" MATERIALS SCIENCE AND ENGINEERING B, ELSEVIER SEQUOIA, LAUSANNE, CH, vol. 34, no. 2, 1 November 1995 (1995-11-01), pages 175-179, XP004000947 ISSN: 0921-5107
- WEI-HAN TAO ET AL: "The humidity sensing properties of organic-inorganic (AMPS/SiO2) hybrid materials" PROCEEDINGS OF IEEE SENSORS 2002. ORLANDO, FL, JUNE 12 - 14, 2002, IEEE INTERNATIONAL CONFERENCE ON SENSORS, NEW YORK, NY : IEEE, US, vol. VOL. 1 OF 2. CONF. 1, 12 June 2002 (2002-06-12), pages 641-646, XP010605175 ISBN: 0-7803-7454-1
- SURI K ET AL: "Gas and humidity sensors based on iron oxide-polypyrrole nanocomposites" SENSORS AND ACTUATORS B, ELSEVIER SEQUOIA S.A., LAUSANNE, CH, vol. 81, no. 2-3, 5 January 2002 (2002-01-05), pages 277-282, XP004329908 ISSN: 0925-4005
- PARK N-G ET AL: "Synthesis and electrochemical properties of V2O5 intercalated with binary polymers" JOURNAL OF POWER SOURCES, ELSEVIER SEQUOIA S.A. LAUSANNE, CH, vol. 103, no. 2, 1 January 2002 (2002-01-01), pages 273-279, XP004312753 ISSN: 0378-7753
- SUKPIROM N ET AL: "PREPARATON OF ORGANIC-INORGANIC NANOCOMPOSITES WITH A LAYERED TITANATE" CHEMISTRY OF MATERIALS, AMERICAN CHEMICAL SOCIETY, WASHINGTON, US, vol. 13, no. 6, 2001, pages 2179-2185, XP001016107 ISSN: 0897-4756
- GOWARD G R ET AL: "Poly(pyrrole) and poly(thiophene)/vanadium oxide interleaved nanocomposites: positive electrodes for lithium batteries" ELECTROCHIMICA ACTA, ELSEVIER SCIENCE PUBLISHERS, BARKING, GB, vol. 43, no. 10-11, 30 April 1998 (1998-04-30), pages 1307-1313, XP004134191 ISSN: 0013-4686
- RUIZ-HITZKY E: "CONDUCTING POLYMERS INTERCALATED IN LAYERED SOLIDS**" ADVANCED MATERIALS, VCH VERLAGSGESELLSCHAFT, WEINHEIM, DE, vol. 5, no. 5, 1 May 1993 (1993-05-01), pages 334-340, XP000368640 ISSN: 0935-9648
- DONG W ET AL: "SOL-GEL SYNTHESIS AND CHARACTERIZATION OF MOLYBDENUM OXIDE/ POLYPYRROLE HYBRIDS" ORGANIC/INORGANIC HYBRID MATERIALS II. SAN FRANCISCO, CA, APRIL 5 - 9, 1999; [MATERIALS RESEARCH SOCIETY SYMPOSIUM PROCEEDINGS. VOL. 576], WARRENDALE, PA : MRS, US, vol. 576, 5 April 1999 (1999-04-05), pages 269-274, XP000897923 ISBN: 978-1-55899-483-6
- SHI-JIE WEN ET AL: "The new phenomenon of lithium electrochemical (de)intercalation in mineral clay materials and their potential application in rechargeable batteries" ACTIVE AND PASSIVE ELECTRONIC COMPONENTS, GORDON AND BREACH, NEW YORK, NY, US LNKD- DOI:10.1155/1994/68435, vol. 16, no. 3-4, 1 January 1994 (1994-01-01), pages 145-152, XP008105772 ISSN: 0882-7516

## Description

### Field

The present invention is concerned with a gas sensor. More specifically, it relates to a novel gas sensor having superior long term stability and superior gas selectivity to a volatile organic compound (VOC) at a temperature of 100°C or less using a particular organic-inorganic hybrid material as a sensor element. Such a gas sensor, for example, does not respond to a flammable gas such as hydrogen and responds selectively to a VOC gas.

The invention is also concerned with methods of producing a gas sensor and to structures suitable for use in gas sensors for volatile organic compounds.

### Background

### Related Work

Sick house syndrome has become a problem as people spend more time indoors with less ventilation. The sick house syndrome is often caused by volatile organic compounds (VOC) released from building materials, furniture, paints, adhesives, and the like. VOC sensors are required to monitor indoor environments. Gas sensors using an n-type semiconductor oxide such as tin oxide, for example, have superior long term stability. Further, they can be miniaturized and do not require any expensive devices for the production and complicated circuits for sensing the change in resistance. Therefore, these sensors have been widely used in practice as household gas leak alarms and the like. Furthermore, research is being carried out related to semiconductor gas sensors for detecting various gases in addition to household gases. Specifically, gas sensors for sensing VOC are being studied. Gas sensors that selectively sense only one gas are required.

However, it is difficult to make semiconductor oxide gas sensors that selectively detect various gases. VOC sensors using a semiconductor oxide known in the related are sensors that sense the total volume of some ten types of VOC. In order to improve the gas selectivity, for example, a method for providing a catalytic layer on a surface of a semiconductor oxide is described in JP 2000-298108 and JP 2000-292397. In addition, a method for coating a surface of a semiconductor oxide with a silica film used as a filer layer is described in JP 10-170464 and JP 2000-275201. Further, a method for adding a noble metal or an oxide to tin oxide is discussed in IEEJ Transactions E, 119, 383-389 (1999) and IEEJ Transactions E, 121, 395-401 (2001).

However, further improvement in gas selectivity is required. In addition, development of VOC gas sensors using a conductive polymer has also been reported. Although the gas selectivity has been improved (see JP 08-015197) compared to that of semiconductor gas sensors, improvement in long term and thermal stability is a major issue.

Furthermore, in a resistance change type sensor using a conductive polymer, in order to avoid polarization, the change in impedance must be measured by applying an alternating current. Accordingly, as compared measuring using direct current, the circuit becomes more complicated.

E. Ruiz-Hitzky, Advanced Materials 1993, vol. 5, pp.334-340 provides a review or several conducting polymers intercalated in layered solids. Paragraph 3 provides a summary of known intercalating conducting polymers in several layered solids. Figure 7 shows poly(p-phenylenevinylene) generation in the interlayer space of MoO₃. This material is prepared according to a method comprising intercatalation of the poly(p-xylylene-aplha-dimethylsulfoxonium) polycation, by ion exchange.

W. Dong et al., Mat. Res Soc. Symp. Proc. 1999, vol. 576, pp. 269-274 discloses the synthesis of MoO₃/PP_{y} hybrid aerogels and xerogels from a molybdenum oxide sol. This publication is silent on the use of said gels for a (VOC or even gas) sensor.

S. Wen et a/., Active and Passive Elec. Comp. 1994, vol 16, pp. 145-152 mentions, in the introductory part, hybrid intercalation composites including MoO₃ + PPY. Especially their use in energy storage is disclosed.

M.A. Bizeto et al., J. Materials Sci. 2001, vol. 37, pp 265-270 discloses a layered element comprising an inorganic-organic layered element, with niobate as the inorganic compound and porphyrin as the organic compound. Also the use as sensor is mentioned.

P. Gomez-Romero et al., Advanced Materials 1997, vol. 9, pp. 144-147 discloses electrodes of a polypyrrole and phoshomolybdenum compounds.

K. Suri et al, Sensors and Actuators, B 81 (2002), 277-282 discloses a CH₄-sensor based on iron oxide-polypyrrole nanocomposites.

Although examples of VOC sensors exist, further improvement is required. High performance gas sensors having superior gas selectivity are required.

### Summary of the invention

It will be significantly advantageous to overcome problems noted above in related art.

There is provided a gas sensor comprising a sensor element including an organic-inorganic hybrid material according to claim 1. The hybrid materials have a layered structure in which an organic compound is intercalated into layers of an inorganic compound.

In a specific enhancement, presence of a gas is detected by a change in resistance.

The inorganic compound comprises molybdenum oxide.

The organic compound comprises a conductive polymer.

In yet another specific enhancement, the gas sensor responds to a volatile organic compound at a temperature in the range of from room temperature (about 20°C) to 80°C.

In still another specific enhancement, the inorganic and organic individual layers have thickness less than 1 nanometer and they are alternately laminated to each other.

Another aspect of the disclosed teachings is a method for manufacturing a gas sensor comprising providing layers of an inorganic compound. An organic compound is intercalated between the layers of the inorganic compound to form a organic-inorganic hybrid material. The hybrid material is used as a sensor element in the gas sensor.

Still another aspect of the disclosed teachings is a method of manufacturing a hybrid organic-inorganic material comprising suspending an inorganic material in distilled water in an argon atmosphere according to claim 9. If necessary, a reducing agent, especially a reducing alkali metal compound such as sodium dithionite or sodium dithionate, and an alkali metal salt, especially a sodium salt, are added and stirred. The inorganic material with hydrated alkali metal ions intercalated between layers of the oxide product is separated, washed and dried. The product is suspended in distilled water. A monomer is added and stirred, e.g. using an ultrasonic homogenizer. A polymerizing agent, usually an oxidizing agent, is added and stirred. The product is separated.

In a specific enhancement, the oxidizing agent is selected from iron chloride, ammonium peroxodisulfate and iron nitrate.

In another specific enhancement, an amount of the monomer is 50 to 200 equivalents to one equivalent of the hydrated sodium ions intercalated material.

More specifically, the amount of conductive polymer is 100 to 150 equivalents to one equivalent of the hydrated sodium ions intercalated material.

In another specific enhancement, the inorganic material is selected from molybdenum oxide, tungsten oxide, vanadium oxide, niobium oxide, ruthenium chloride, iron oxychloride and molybdenum sulfide.

In yet another specific enhancement, the conductive polymer is selected from a group consisting of polypyrrole, polyaniline, polythiophene and polyethylene oxide.

### Description of the drawings

The disclosed teachings will become more apparent by describing in detail examples and embodiments thereof with reference to the attached drawings in which:

Fig. 1 is a schematic view showing one example of a crystal structure of a gas sensor material according to the disclosed teachings.

Fig. 2 is a graph showing x-ray diffraction patterns of [Na(H₂O)₅]ₓMoO₃ and PPy_{y}MoO₃ described in Example 1.

Fig. 3 is a graph showing infrared spectra of [Na(H₂O)₅]ₓMoO₃, PPy_{y}MoO₃, and simple polypyrrole (PPy) prepared by a chemical polymerization method described in Example 1.

Fig. 4 is a graph showing the results of thermogravimetric analysis of PPy_{y}MoO₃ and simple polypyrrole (PPy) formed by a chemical polymerization method described in Example 1.

Fig. 5 is a graph showing sensor sensitivity of a sensor described in Example 1 to a methanol gas.

Fig. 6 is a graph showing an x-ray diffraction pattern of PPy_{y}MoO₃ described in Example 2.

### Detailed description

### Synopsis

A new sensing principle which does not depend on the sensing principle of the semiconductor oxide sensor described above is disclosed herein. A novel sensor using a hybrid material obtained by nanometer-level integration of an organic material and an inorganic material is provided. The organic material is used to recognize the target gas molecules. The inorganic material is used to convert chemical information (representing whether a gas to be sensed is present or not) to an electrical signal. This information is output.

When an organic compound and an inorganic compound are allowed to perform different functions and are integrated together on a nano-scale, a sensor can be made which has both high sensitivity of the organic compound and high stability of the inorganic compound and which can easily output a signal outside. A hybrid sensor, which is formed by a combination of an appropriate metal oxide and a conductive polymer, responds selectively to a VOC gas as the resistance is changed. It is also stable as compared to a sensor composed of only a conductive polymer.

### Illustration of the concepts underlying the disclosed teachings

The disclosed teachings are described in more detail herein.

A metal oxide having a layered structure is used as the inorganic compound. An organic polymer is intercalated between these layers. The organic-inorganic hybrid sensor is formed by nanometer-level integration. The combination between the organic compound and the inorganic compound needs to be appropriately selected. For example, when a combination of an organic compound and an inorganic compound, which have different types of carriers responsible for conductivity, is selected, charge transfer occurs between these two compounds. As a result, conductivity is imparted to the hybrid sensor.

In the sensor described above, for example, when incorporation of a VOC gas into the organic polymer layers causes the change in the interlayer distance, the charge transfer is influenced. This changes the electrical resistance of the sensor. A hybrid material obtained by the combination of an inorganic compound having conductivity and an organic compound having insulating properties exhibits conductivity. However, since the charge transfer does not occur, the sensing function based on the principle described above cannot be expected. The sensing can be achieved only when the inorganic compound (which forms the disclosed hybrid sensor) has a layered structure and is provided with conductivity by the charge transfer from the organic compound.

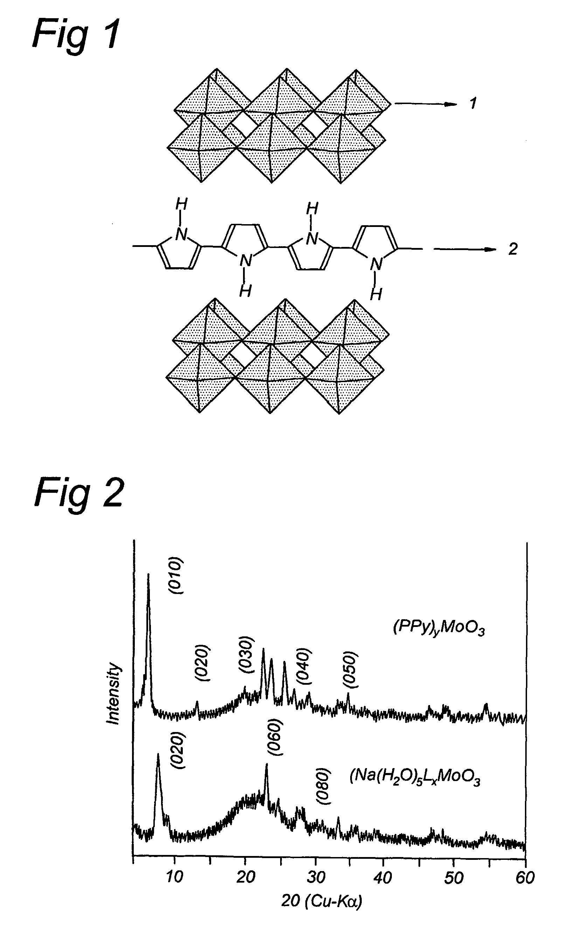

An interlayer compound used for a gas sensor comprises an inorganic compound having a layered structure and an organic compound intercalated between the inorganic layers. As one example, Fig. 1 shows a schematic view of a crystal structure of an interlayer compound. In this structure, molybdenum oxide (shown in the figure as layer 1) is the inorganic compound with a layered structure and polypyrrole (shown in the figure as layer 2) (PPy) is the organic compound.

A layered structure (hereinafter, this material will be described as PPy_{y}MoO₃) is formed in which polypyrrole is intercalated between layers of molybdenum oxide. Although molybdenum oxide itself is an insulating material, it is turned into an n-type semiconductor when electrons are supplied thereinto. On the other hand, as in the case described above, polypyrrole is turned into a p-type semiconductor when emitting electrons. Accordingly, conductivity is generated in PPy_{y}MoO₃ by the charge transfer from polypyrrole to molybdenum oxide.

Although PPy_{y}MoO₃ is synthesized in the form of a powder, it can be easily molded by press molding. Hence, a sensor element can be formed by providing electrodes on the press-molded body thus formed.

PPy_{y}MoO₃ can be prepared by a two-stage reaction. First, [Na(H₂O)₅]ₓMoO₃ is synthesized in which hydrated sodium ions are intercalated between layers of molybdenum oxide. In this stage, molybdenum oxide is suspended in distilled water in an argon atmosphere, and sodium dithionite and sodium molybdate are added thereto, followed by stirring. The reaction time is generally 5 minutes to one hour, and preferably 10 to 30 minutes. A reaction product is separated, washed, and dried, thereby obtaining [Na(H₂O)₅]ₓMoO₃.

Next, at the second stage reaction, [Na(H₂O)₅]ₓMoO₃ is suspended in distilled water, and pyrrole is added thereto, followed by sufficient stirring. Subsequently, an oxidizing agent is added to the mixture thus formed and the mixture is then further stirred. A reaction product is separated, washed, and dried, thereby obtaining PPy_{y}MoO₃. The oxidizing agent used at this stage is not specifically limited as long as it can oxidize pyrrole. Trivalent iron salts such as iron chloride and iron nitrate and peroxodiulfates such as ammonium peroxodisulfate, are examples of oxidizing agents that can be used. The amount of pyrrole added is generally 50 to 200 equivalents and preferably 100 to 150 equivalents with respect to one equivalent of [Na(H₂O)₅]ₓMoO₃. The amount of the oxidizing agent added is generally 1 to 3 equivalents and preferably 1.5 to 2 equivalents with respect to one equivalent of [Na(H₂O)₅]ₓMoO₃.

In general, an electrical resistance change type sensor composed of an n-type semiconductor oxide responds to a VOC gas as that the resistance is decreased. However, the gas sensor disclosed herein responds to a VOC gas such that a sensor resistance is increased. In particular, among VOC gases, the sensor responds selectively to polar molecules such as aldehydes and alcohols. Compared to a non-polar molecule, a polar molecule generates a strong interaction with polypyrrole having positive charge, and hence it is believed that a polar molecule infiltrates a polypyrrole layer having the crystal structure shown in Fig. 1. As a result, the interlayer distance is increased, and the charge transfer is suppressed, resulting in increase in resistance of the sensor. That is, such a gas sensor exhibits a selective and particular response to a gas having polarity among VOC gases.

Furthermore, since the disclosed sensor exhibits a response at room temperature, a heating mechanism and the like are not particularly necessary. In addition, since polarization is not generated unlike a simple conductive polymer, resistance measurement can be performed using a direct current. Therefore, a complicated measuring circuit using an alternating current is not necessary. Accordingly, the sensor unit can be miniaturized.

In addition, according to the results of a thermogravimetric analysis performed on the disclosed organic-inorganic hybrid material, the decomposition temperature of polypyrrole is increased by approximately 50°C as compared to pure polypyrrole which is formed separately. The thermal stability is improved by hybridization.

Furthermore, the long term stability is also superior. Even when being held in the air for a half year or more after the formation, degradation in conductivity is not observed. A wearable VOC monitoring device cane be made using the sensor disclosed herein.

The disclosed techniques in which molybdenum oxide and polypyrrole are used may also be applied to a layered inorganic compound and a conductive polymer other than polypyrrole. Examples of the conductive polymer that can be used include polypyrroles, polyanilines, polythiophenes, polyacetylenes and their substituted analogs. However, they are not limited to those mentioned above. Materials that produce the same effect as that of the above compounds may also be used.

### Examples

### Example 1

(1) Formation of PPy_{y}MoO₃

Molybdenum oxide MoO₃ powder (1g, 6.9 mmol) was added to 50 ml of distilled water and was stirred for 30 minutes at room temperature while an argon gas was being bubbled. To the mixture thus formed, after sodium dithionite Na₂S₂O₄ (0.35 g, 2.0 mmol) and sodium molybdate Na₂MoO₄.2H₂O (12 g, 49.6 mmol) were added, stirring was performed for 10 minutes at room temperature while an argon gas was being bubbled. The color of the molybdenum oxide powder quickly changed from white into deep blue upon adding sodium dithionite and sodium molybdate, and hence it was confirmed that molybdenum oxide was reduced. After stirring was performed, a reaction product was processed by vacuum filtration and was then washed with distilled water, and spontaneous drying was then performed in the air. Based on the results of the thermogravimetric analysis, it was found that x=0.17.

Next, [Na(H₂O)₅]ₓMoO₃ (0.1 g, 0.61 mmol) formed by the method described above was suspended in 40 ml of distilled water, and after pyrrole (5.8 ml, 89.22 mmol) was added thereto, the mixture thus formed was processed using a ultrasonic homogenizer for 3 minutes. To this mixture thus processed, iron chloride FeCl₃ (0.146 g, 0.90 mmol) was added as an oxidizing agent and was then stirred for 10 minutes at room temperature. In addition, after 40 ml of ethanol was added and was then stirred for 20 minutes, a reaction product was processed by vacuum filtration, washed with ethanol, and spontaneously dried in the air, thereby obtaining PPy_{y}MoO₃. An x-ray diffraction pattern of the PPy_{y}MoO₃ thus formed by the above process is shown in Fig. 2 together with that of [Na(H₂O)₅]ₓMoO₃ for comparison. In both samples, the crystallographic b axis is perpendicular to the molybdenum oxide layers, and by observing the (0k0) peak of the x-ray diffraction pattern, the change in interlayer distance could be understood.

Since the (0k0) peak of PPy_{y}MoO₃ was shifted to the low angle side as compared to that of [Na(H₂O)₅]ₓMoO₃, it is understood that the interlayer distance of PPy_{y}MoO₃ was increased. The increase in interlayer distance of PPy_{y}MoO₃ was 0.63 nm as compared to that of simple molybdenum oxide, and this shows that polypyrrole was intercalated between the layers of molybdenum oxide.

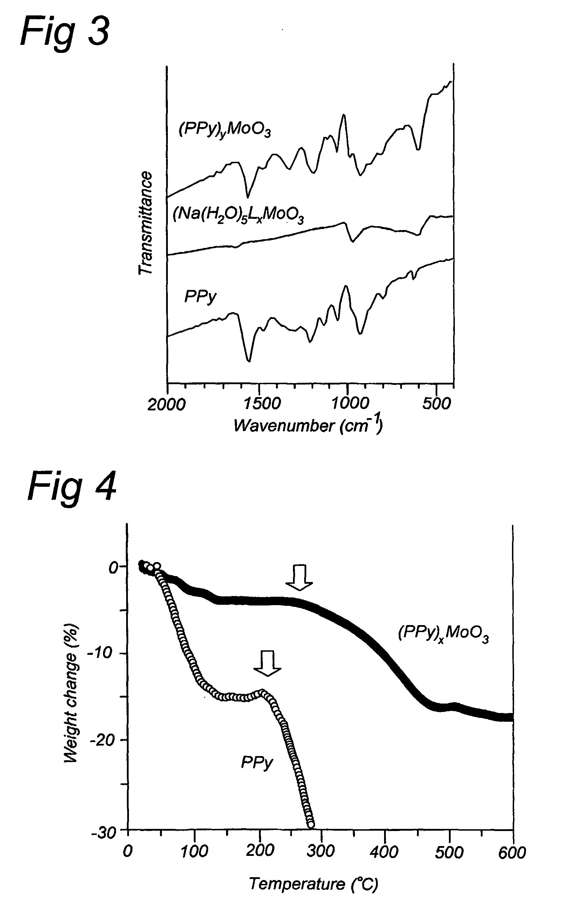

In Fig. 3, infrared spectra of [Na(H₂O)₅]ₓMoO₃, PPy_{y}MoO₃, and polypyrrole (PPy) prepared by a chemical polymerization method are shown. In PPy_{y}MoO₃, absorption of molybdenum oxide, which was also observed in [Na(H₂O)₅]ₓMoO3, and absorption of polypyrrole were both observed, and hence it was confirmed that a layered compound composed of molybdenum oxide and polypyrrole intercalated between the layers thereof was formed. In Fig. 4, the result of the thermogravimetric analysis of PPy_{y}MoO₃ is shown together with that of the polypyrrole formed by a chemical polymerization method for comparison. Temperatures indicated by individual arrows are decomposition temperatures of polypyrroles. The decomposition temperature of the layered compound PPy_{y}MoO₃ was higher than that of pure polypyrrole by approximately 60°C, and this shows that the hybridization with an inorganic compound is effective to improve the thermal stability of an organic polymer. In this figure, the decrease in weight up to approximately 100°C was caused by adsorbed water. In addition, from the decrease in weight caused by decomposition of pyrrole, as the y value of PPy_{y}MoO₃, y=0.3 was obtained.

(2) Evaluation of Electrical Properties and Sensor Properties

A sample obtained by performing press molding for PPy_{y}MoO₃ powder thus formed was provided with electrodes using a silver paste, thereby forming a sensor element. The electrical resistivity of the press-molded body measured by a DC four-probe method was 9.6 Ω•cm at room temperature. The electrical resistivity of a press-molded body of powdered [Na(H₂O)₅]ₓMoO₃ measured in the same manner as that described above was 3,200 Ω •cm, and hence by intercalating polypyrrole between the layers, the electrical resistivity was decreased by two orders of magnitude or more. This shows that the polypyrrole layer has conductivity as is the molybdenum oxide layer, and that the charge transfer is realized between the molybdenum oxide layer and the polypyrrole layer.

Sensor properties of the PPy_{y}MoO₃ sensor to hydrogen, carbon monoxide, carbon dioxide, and methane gas were evaluated by the change in electrical resistance when the sensor was exposed to the individual gases. The concentrations of the gases for evaluation were all set to 3% (balanced with air), and the measurement temperature was set to room temperature and 100°C. The measurement was performed by allowing clean air (one minute), a sample gas (three minutes), and clean air (16 minutes) to flow in that order, and the total 20 minutes was regarded as one cycle.

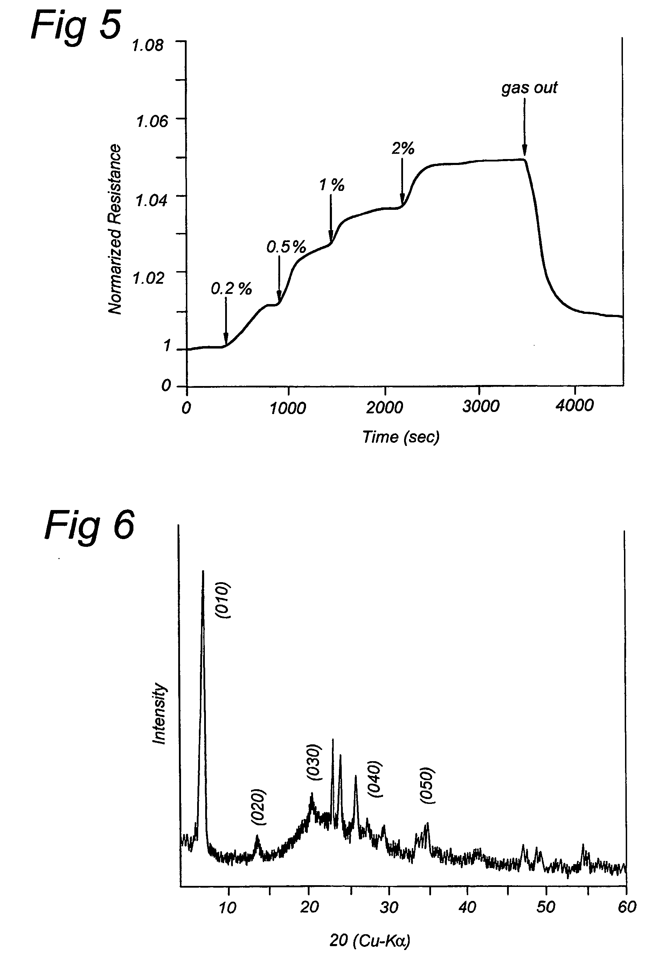

According to the measurement results, the PPy_{y}MoO₃ sensor did not respond to these gases. On the other hand, the measurement result of the sensor properties to methanol is shown in Fig. 5. For the measurement of methanol, the sensor was placed in an airtight type chamber, and after a liquid methanol was supplied to a heater provided in the chamber for evaporation to obtain a predetermined concentration, the change in resistance of the sensor was measured.

The vertical axis in the figure indicates resistance normalized by the initial resistance. A response to methanol was shown, in which the resistance of the sensor was increased. The sensor sensitivity (R_{gas}/R₀: R₀ indicates the initial resistance, and R_{gas} indicates the resistance in a gas atmosphere) to various VOC gases at a concentration of 1,000 ppm at room temperature is shown in Table 1. The sensor hardly responded to toluene and benzene but selectively responded to aldehydes, alcohols, and a chlorinated gas. Among those mentioned above, particularly high sensitivity was shown to formaldehyde. This fact shows that the disclosed sensor hardly responds to flammable gases such as hydrogen and has high selectivity to VOC gasses.

**Table 1**

| Sensor Sensitivity of PPy_{y}MoO₃ Sensor at Room Temperature to Various VOC Gases at a Concentration of 1,000 ppm | |
|---|---|
| Gas | Sensor Sensitivity (R_{gas}/R₀), % |
| Formaldehyde | 6.0 |
| Methanol | 0.8 |
| Ethanol | 0.7 |
| Chloroform | 2.0 |
| Acetone | 0.2 |
| Toluene | <0.1 |
| Benzene | <0.1 |

### Example 2

A PPy_{y}MoO₃ sensor was formed in a manner similar to that described in Example 1 except that the oxidizing agent used for intercalation reaction of polypyrrole into [Na(H₂O)₅]ₓMoO₃ was ammonium peroxodisulfate (NH₄)₂S₂O₈. The x-ray diffraction pattern of a formed powder is shown in Fig. 6. As was the case of Example 1, it was confirmed that an interlayer compound of molybdenum oxide and polypyrrole intercalated between the layers thereof was formed. When the sensor properties were measured, the similar results as those in Example 1 were obtained.

### Example 3

[Na(H₂O)₅]ₓMoO₃ was formed in a manner similar to that described in Example 1 except that the reaction time for forming [Na(H₂O)₅]ₓMoO₃ was set to three hours. According to the x-ray diffraction measurement for the [Na(H₂O)₅]ₓMoO₃ thus formed, the generation of impurities was observed as the reaction time was increased. From the results thus obtained, it was found that the reaction time for forming [Na(H₂O)₅]ₓMoO₃ is preferably set to 30 minutes or less.

The following advantages are realized using various aspects of the disclosed teachings:

(1) A novel gas sensor based on a new sensing principle which does not depend on that of a conventional semiconductor oxide sensor is provided. A manufacturing technique of the gas sensor described is also provided.

(2) A novel gas sensor based on the following sensing principle is provided. That is, in an interlayer compound according to claim 1 made of an inorganic compound and an organic compound alternately laminated to each other, the interlayer distance is changed by a gas which is to be sensed, and as a result, the sensor resistance is controlled.

(3) According to the disclosed manufacturing method according to claim 9, a novel gas sensor composed of an interlayer compound can be manufactured, in which an inorganic compound and an organic compound are alternately laminated to each other.

(4) The gas sensor thus formed hardly responds to flammable gases such as hydrogen but responds selectively to VOC gases.

(5) The gas sensor thus formed is useful as a VOC sensor.

Other modifications and variations to the invention will be apparent to those skilled in the art from the foregoing disclosure and teachings. Thus, while only certain embodiments of the invention have been specifically described herein, it will be apparent-that numerous modifications may be made thereto.

## Claims

1. A gas sensor for determining volatile organic compounds comprising a sensor element including an organic-inorganic hybrid material, said sensor element having layers of an inorganic compound and an organic compound intercalated between the layers of an inorganic compound, wherein the inorganic compound comprises molybdenum oxide, and wherein the organic compound comprises a conductive polymer.

2. The gas sensor of claim 1, wherein presence of a gas is detected by a change in resistance.

3. The gas sensor of any one of claims 1-2, wherein the organic compound comprises a polypyrrole.

4. The gas sensor of any one of claims 1-3, wherein the gas sensor responds to a volatile organic compound at a temperature in the range of from room temperature to 80°C.

5. The gas sensor of any one of claims 1-4, wherein the inorganic and organic individual layers have thickness less than 1 nanometer and they are alternately laminated to each other.

6. Use of an organic-inorganic hybrid material as defined in anyone of claims 1-5 in a gas sensor for sensing volatile organic compound (VOC).

7. The use according to claim 6, wherein presence of a VOC gas is detected by a change in resistance.

8. The use according to of any one of claims 6-7, wherein the gas sensor responds to a volatile organic compound at a temperature in the range of from room temperature to 80°C.

9. A method of manufacturing a hybrid organic-inorganic material comprising:
a) suspending MoO₃ in distilled water in an inert atmosphere;
b)adding sodium molybdate and sodium dithionite, followed by stirring;
c) separating, washing and drying the reaction product, thereby obtaining [Na(H₂O)₅]ₓMoO₃];
d) suspending the [Na(H₂O)₅]ₓMoO₃] in distilled water;
e) adding an organic monomer and homogenizing;
f) adding an oxidizing agent as polymerizing agent, and stirring; and
g) separating the product of step f to provide an organic-inorganic hybrid material, having layers of an inorganic compound and an organic compound intercalated between the layers of an inorganic compound, wherein the inorganic compound comprises molybdenum oxide, and wherein the organic compound comprises a conductive polymer.

10. The method of claim 9, wherein the oxidizing agent is selected from the group consisting of iron chloride, ammonium peroxodisulfate and iron nitrate.

11. The method of claim 9 or 10, wherein an amount of the conductive polymer is 50 to 200 equivalents, preferably 100 to 150 equivalents to one equivalent of the inorganic material is added.

12. The method of claim 11, wherein the conductive polymer is selected from the group consisting of polypyrrole, polyaniline, polythiophene and polyacetylene, and their substituted analogs.

## Patentansprüche

1. Gassensor zum Bestimmen von flüchtigen organischen Verbindungen umfassend ein Sensorelement einschließlich eines organischen-anorganischen, hybriden Materials, wobei das Sensorelement Schichten einer anorganischen Verbindung und eine organische Verbindung aufweist, die zwischen den Schichten von einer anorganischen Verbindung eingebunden ist, wobei die anorganische Verbindung Molybdänoxid umfasst und wobei die organische Verbindung ein leitendes Polymer umfasst.

2. Gassensor nach Anspruch 1, wobei die Präsenz von einem Gas durch eine Änderung des Widerstandes entdeckt wird.

3. Gassensor nach einem der vorhergehenden Ansprüche 1-2, wobei die organische Verbindung ein Polypyrrol umfasst.

4. Gassensor nach einem der vorhergehenden Ansprüche 1-3, wobei der Gassensor auf eine flüchtige organische Verbindung bei einer Temperatur, die zwischen Raumtemperatur und 80 °C liegt, reagiert.

5. Gassensor nach einem der vorhergehenden Ansprüche 1-4, wobei die anorganischen und organischen individuellen Schichten eine Dichte von weniger als 1 Nanometer aufweisen und wechselweise aufeinander geschichtet sind.

6. Verwendung eines organischen-anorganischen, hybriden Materials, das in einem der vorhergehenden Ansprüche 1-5 definiert ist, in einem Gassensor zum Erfassen einer flüchtigen organischen Verbindung (volatile organic compound, VOC).

7. Verwendung nach Anspruch 6, wobei die Gegenwart eines VOC-Gases durch eine Änderung des Widerstandes entdeckt wird.

8. Verwendung nach einem der vorhergehenden Ansprüche 6-7, wobei der Gassensor auf eine flüchtige organische Verbindung bei einer Temperatur, die zwischen Raumtemperatur und 80 °C liegt, reagiert.

9. Verfahren zur Herstellung eines hybriden, organischen-anorganischen Materials, umfassend:
a) Aussetzen von MoO₃ in destilliertem Wasser in einer inerten Atmosphäre;
b) Zugeben von Natriummolybdat und Natriumdithionit, gefolgt von Rühren;
c) Trennen, Waschen und Trocknen des Reaktionsproduktes, wodurch [Na(H₂O)₅]ₓMoO₃] erhalten wird;
d) Aussetzen von [Na(H₂O)₅]ₓMoO₃] in destilliertem Wasser;
e) Zugeben von einem organischen Monomer und Homogenisieren;
f) Zugeben von einem oxydierendem Wirkstoff als polymerisierenden Wirkstoff und Rühren und
g) Trennen des Produkts von Schritt f zur Bereitstellung eines organischen-anorganischen, hybriden Materials, was Schichten von einer anorganischen Verbindung und eine organische Verbindung, die zwischen den Schichten einer anorganischen Verbindung eingebunden ist, aufweist, wobei die anorganische Verbindung Molybdän Oxid umfasst und wobei die organische Verbindung ein leitendes Polymer umfasst.

10. Verfahren nach Anspruch 9, wobei der oxydierende Wirkstoff aus einer Gruppe bestehend aus Eisenchlorid, Ammoniumperoxodisulfat und Eisennitrat gewählt wird.

11. Verfahren nach Anspruch 9 oder 10, wobei eine Menge des leitenden Polymers als 50 bis 200 Entsprechungen, vorzugsweise 100 bis 150 Entsprechungen zu einem Gegenwert des anorganischen Materials hinzugefügt wird.

12. Verfahren nach Anspruch 11, wobei das leitende Polymer aus einer Gruppe bestehend aus Polypyrrol, Polyanilin, Polythiophen und Polyacetylen und deren substituierten Nachbildungen gewählt wird.

## Revendications

1. Capteur de gaz pour la détermination des composés organiques volatils comportant un élément capteur comprenant un matériau hybride organique/inorganique, ledit élément capteur présentant des couches d'un composé inorganique et un composé organique intercalé entre les couches d'un composé inorganique, dans lequel le composé inorganique comprend de l'oxyde de molybdène, et dans lequel le composé organique comprend un polymère conducteur.

2. Capteur de gaz selon la revendication 1, dans lequel la présence d'un gaz est détectée par un changement de résistance électrique.

3. Capteur de gaz selon l'une quelconque des revendications 1 à 2, dans lequel le composé organique comprend du polypyrrole.

4. Capteur de gaz selon l'une quelconque des revendications 1 à 3, dans lequel le capteur de gaz répond à un composé organique volatil à une température comprise entre la température ambiante et 80°C.

5. Capteur de gaz selon l'une quelconque des revendications 1 à 4, dans lequel les couches individuelles inorganiques et organiques présentent une épaisseur inférieure à 1 manomètre et sont alternativement stratifiées les unes aux autres.

6. Utilisation d'un matériau hybride organique/inorganique telle que définie dans l'une quelconque des revendications 1 à 5 dans un capteur de gaz pour détecter un composé organique volatil (COV).

7. Utilisation selon la revendication 6, dans laquelle la présence d'un gaz COV est détectée par un changement de résistance électrique.

8. Utilisation selon l'une quelconque des revendications 6 à 7, dans laquelle le capteur de gaz répond à un composé organique volatil à une température comprise entre la température ambiante et 80°C.

9. Procédé de fabrication d'un matériau hybride organique/inorganique consistant :
a) à mettre en suspension du MoO₃ dans de l'eau distillée dans une atmosphère inerte ;
b) à ajouter du molybdate de sodium et du dithionite de sodium, puis à mélanger ;
c) à séparer, à laver et à sécher le produit de réaction, afin d'obtenir du [Na(H₂O)₅]ₓMoO₃] ;
d) à mettre en suspension le [Na(H₂O)₅]ₓMoO₃] dans de l'eau distillée ;
e) à ajouter un monomère organique et à homogénéiser ;
f) à ajouter un agent oxydant en tant qu'agent de polymérisation, puis à mélanger ; et
g) à séparer le produit de l'étape f pour fournir un matériau hybride organique/inorganique, présentant des couches d'un composé inorganique et un composé organique intercalé entre les couches d'un composé inorganique, dans lequel le composé inorganique comprend de l'oxyde de molybdène et dans lequel le composé organique comprend un polymère conducteur.

10. Procédé selon la revendication 9, dans lequel l'agent oxydant est sélectionné dans le groupe constitué de chlorure de fer, de peroxodisulfate d'ammonium et de nitrate de fer.

11. Procédé selon la revendication 9 ou 10, dans lequel on ajoute une quantité du polymère conducteur comprenant de 50 à 200 équivalents, de préférence de 100 à 150 équivalents, par rapport à un équivalent du matériau inorganique.

12. Procédé selon la revendication 11, dans lequel le polymère conducteur est sélectionné dans le groupe constitué de polypyrrole, de polyaniline, de polythiophène et de polyacétylène, et de leurs analogues substitués.
